# FASCICULE DE BREVET EUROPEEN

(11) **EP 2 536 413 B1**
(45) Date de publication et mention de la délivrance du brevet: **01.06.2016**
(21) Numéro de dépôt: 11712922.1
(22) Date de dépôt: 16.02.2011
(51) Int. Cl.: A61L 15/18, A61L 15/28, A61L 15/44, A61L 15/60, A61F 13/00, A61K 31/7016, A61K 31/702

(54) **UTILISATION D'OLIGOSACCHARIDES POLYSULFATES SYNTHETIQUES COMME AGENTS DE DETERSION D'UNE PLAIE**
VERWENDUNG VON SYNTHETISCHEN POLYSULFATIERTEN OLIGOSACCHARIDE ALS MITTEL FÜR DIE WUND REINIGUNG
USE OF SYNTHETIC POLYSULFATED OLISACCHARIDES AS AN AGENT FOR WOUND DETERSION

(30) Priorité: 17.02.2010 FR 1051142
(43) Date de publication de la demande: 26.12.2012
(73) Titulaire: URGO RECHERCHE INNOVATION ET DEVELOPPEMENT, 21300 Chenôve (FR)
(72) Inventeur: APERT, Laurent, F-21000 Dijon (FR); LAURENSOU, Christelle, F-21000 Dijon (FR); NICOT, Dominique, F-21000 Dijon (FR)
(74) Mandataire: Hubert, Philippe
(86) Numéro de dépôt international: PCT/FR2011/050329
(87) Numéro de publication internationale: WO 2011/101594

(56) Documents cités:
- EP-A1- 1 764 104
- EP-A2- 0 230 023
- WO-A1-88/09347
- WO-A1-94/00476
- WO-A1-95/14483
- WO-A1-95/34313
- WO-A1-99/18974
- WO-A2-2004/062674
- US-A- 4 912 093
- US-A- 5 246 708
- US-A1- 2007 037 776
- US-A1- 2008 299 147

## Description

La présente invention a pour objet l'utilisation comme agent de détersion, d'au moins un composé choisi parmi les oligosaccharides polysulfatés synthétiques ayant 1 à 4 unités oses, leurs sels et complexes.

Plus précisément, la présente invention concerne l'utilisation d'au moins un tel composé comme agent de dégradation des matrices de fibrine qui entrent dans la composition du tissu fibrineux. Notamment, la présente invention concerne la préparation d'une composition destinée à être utilisée dans le cadre de la détersion assistée.

La cicatrisation d'une plaie est un phénomène biologique naturel, les tissus humains et animaux étant capables de réparer des lésions localisées par des processus de réparation et de régénération qui leur sont propres.

La cicatrisation naturelle d'une plaie se déroule selon trois phases successives, chacune des ces phases étant caractérisée par des activités cellulaires spécifiques qui font progresser le processus de réparation selon des séquences chronologiques précises : la phase de détersion, la phase de bourgeonnement ou granulation et la phase d'épithélialisation.

Immédiatement après un traumatisme, l'organisme réagit en mettant en oeuvre des phénomènes vasculaires et inflammatoires afin de stopper tout risque d'hémorragie et de protéger la plaie contre les risques d'infection.

Ces phénomènes conduisent à la formation d'une matrice à base de fibrine qui va participer à l'arrêt de l'hémorragie. Cette matrice permet aussi une fermeture provisoire et grossière de la plaie.

Afin d'amorcer l'étape suivante de bourgeonnement, l'organisme organise en parallèle la dégradation de cette matrice de fibrine pour laisser place à la matrice extracellulaire synthétisée par les fibroblastes qui sont des acteurs fondamentaux de la phase de bourgeonnement. C'est principalement cette phase d'élimination de la matrice de fibrine et des divers débris présents dans la plaie que l'on désigne sous le terme de détersion.

La phase de détersion est essentielle dans le processus de cicatrisation. Sa mise en oeuvre et sa vitesse de réalisation sont déterminantes pour la réussite et la rapidité du processus de cicatrisation.

Toutefois, les capacités de la détersion naturelle peuvent être insuffisantes lorsque le traumatisme est important ou lorsque le patient souffre de pathologies parallèles, comme les pathologies veineuses ou le diabète. On observe ainsi, dans ces cas, un allongement considérable de la durée de la phase de détersion conduisant à des plaies chroniques difficiles à soigner, comme par exemple l'ulcère de jambe.

On distingue principalement deux types de plaies.

Les plaies qui présentent des plaques de tissus noires et plus ou moins dures, désignés communément sous le terme de "tissus nécrotiques".

Les plaies qui présentent des tissus mous et jaunâtres, désignés communément sous le terme de "tissu fibrineux" ou "fibrine jaune".

Dans le cas de plaies pour lesquelles le processus de détersion naturelle est insuffisant, telles les plaies chroniques, il est nécessaire d'éliminer les tissus nécrotiques et/ou fibrineux. L'élimination de ces tissus nécrotiques et fibrineux, qui peut être réalisée à l'aide de différentes techniques, est désignée couramment sous le terme de "détersion assistée" par opposition à la détersion naturelle.

Selon la technique utilisée, la détersion assistée peut être qualifiée de détersion mécanique ou chirurgicale, de détersion enzymatique, de détersion autolytique ou de détersion biologique.

L'objectif de la détersion assistée est de nettoyer la plaie, en éliminant les tissus nécrotiques et/ou fibrineux qui sont à l'origine des risques d'infection et peuvent être source de douleur ou d'odeurs désagréables, et tout en sauvegardant le maximum de tissus sains présents dans la plaie.

Toutefois, aucune des techniques de détersion actuellement utilisées n'est optimale et toutes ces techniques présentent de nombreux inconvénients.

La détersion chirurgicale ou mécanique est une technique rapide qui consiste à découper les tissus nécrotiques et/ou fibrineux, soit à l'aide d'un bistouri, de pinces, de ciseaux ou d'une curette de Brock, soit à l'aide d'appareils sophistiqués par jets d'eau sous pression ou excision au laser. Cette technique est réalisée au lit du patient ou en milieu chirurgical selon la gravité de la plaie.

Cependant, cette technique est souvent douloureuse et peut entraîner des saignements et parfois même une hémorragie. Elle est alors traumatisante pour le patient. Elle nécessite aussi couramment une médication antalgique au préalable qui allonge la durée des soins.

La détersion enzymatique est réalisée au moyen d'enzymes protéolytiques telles la streptokinase, la trypsine ou la collagénase.

Cependant, l'utilisation de ces enzymes est loin d'être optimale. En effet, ces enzymes peuvent être inactivées par l'utilisation locale d'antiseptiques et leur activité peut être limitée par les inhibiteurs sériques présents dans les exsudats de la plaie. De plus, certaines de ces enzymes présentent des demi-vies très courtes et doivent être renouvelées souvent. Ces enzymes peuvent en outre entraîner des sensations douloureuses lors de leur application, des réactions de sensibilisation, d'allergie voire même créer des érythèmes locaux.

Dans le cas de la détersion biologique, appelée aussi asticothérapie, la dégradation des tissus nécrotiques et/ou fibrineux est réalisée à l'aide de larves de mouches qui se nourrissent exclusivement de tissus morts.

Cette technique présente, elle aussi, plusieurs inconvénients comme un prurit local intense, un certain degré d'inflammation et une sensation de brûlure locale. En outre, cette technique est contre-indiquée chez les patients présentant des troubles de la coagulation ou dont les plaies sont mal vascularisées ou à proximité des organes vitaux ou de gros vaisseaux. Enfin, quoique très efficace, l'utilisation de la détersion biologique se heurte aussi à des difficultés ou des freins psychologiques du patient qui voit ou ressent la présence des larves dans sa plaie.

La détersion autolytique consiste à déposer sur la plaie des pansements absorbants à base de fibres gélifiantes qui, en créant un milieu humide, favorisent le rôle détersif naturel par les macrophages et les polynucléaires neutrophiles tout en permettant de ramollir le tissu fibrineux et d'absorber les débris. Un tel pansement à base de carboxyméthylcellulose est par exemple commercialisé par la société CONVATEC sous la dénomination AQUACEL^{®}.

Toutefois, leur action est lente et insuffisante pour éliminer en grande quantité le tissu fibrineux. Il est donc nécessaire de compléter la détersion autolytique par une autre technique qui est très souvent la détersion mécanique.

Comme on dispose de composés qui permettent d'accélérer ou de favoriser les autres phases du processus de cicatrisation, il serait donc souhaitable de disposer de composés ou de mélanges de composés exempts de substances allergisantes comme les enzymes, qui permettraient de dégrader les tissus nécrotiques et/ou fibrineux pour optimiser cette phase de détersion.

L'utilisation de tels composés permettraient en effet :
- d'une part, de ne pas recourir aux techniques traumatisantes sur le plan physique ou psychologique telles que la détersion mécanique ou l'asticothérapie ; et
- d'autre part, d'optimiser la détersion autolytique en incorporant ces composés dans des pansements absorbants, en fournissant ainsi un produit permettant la dégradation des tissus nécrotiques et/ou fibrineux et l'absorption de ces derniers en une seule étape.

Dans ce contexte, la présente invention a pour but de résoudre le nouveau problème technique consistant en la fourniture de tels composés qui permettent la détersion de la plaie par dégradation des matrices de fibrine qui composent les tissus nécrotiques et/ou les tissus fibrineux.

Il a été découvert, de façon tout à fait surprenante et inattendue, que les oligosaccharides polysulfatés synthétiques ayant 1 à 4 unités oses, leurs sels et complexes, et en particulier le sel de potassium du sucrose octasulfate permettent de dégrader les matrices de fibrine et peuvent donc être utiles comme agent de détersion.

Ainsi, selon un premier aspect, la présente invention a pour objet une composition comprenant au moins un composé choisi parmi les oligosaccharides polysulfatés synthétiques ayant 1 à 4 unités oses, leurs sels et complexes, pour son utilisation comme agent de détersion d'une plaie.

Les oligosaccharides susceptibles d'être utilisés dans le cadre de la présente invention sont des polymères synthétiques formés de 1 à 4 unités d'oses, et de préférence de 1 ou 2 unités d'oses, généralement liées entre elles par liaison glycosidique alpha ou bêta. En d'autres termes, il s'agit de mono, di, tri ou tétrasaccharides, et de préférence de mono ou dissacharides.

Il n'y a pas de limitation particulière concernant la nature des unités oses de ces polysaccharides. De préférence, il s'agira de pentoses ou d'hexoses.

A titre d'exemple de monosaccharide, on peut citer le glucose, le galactose ou le mannose.

A titre d'exemple de disaccharide, on peut citer le maltose, le lactose, le sucrose ou le tréhalose.

A titre d'exemple de trisaccharide, on peut citer le mélézitose.

A titre d'exemple de tétrasaccharide, on peut citer le stachyose.

De préférence, l'oligosaccharide est un disaccharide, de préférence encore le sucrose.

Par l'expression "oligosaccharide polysulfaté", on entend désigner ici un oligosaccharide dont au moins deux, et de préférence tous les groupes hydroxy de chaque ose ont été substitués par un groupe sulfate.

De préférence, l'oligosaccharide polysulfaté est le sucrose octasulfate.

Les oligosaccharides polysulfatés utilisés dans le cadre de la présente invention peuvent se présenter sous forme de sels ou complexes.

A titre d'exemple de sels, on peut citer les sels de métal alcalin tel que le sel de sodium, de calcium ou de potassium ; un sel d'argent ; ou encore un sel d'acide aminé.

A titre d'exemple de complexes, on peut citer les complexes d'hydroxyaluminium.

Dans le cadre de la présente invention, des composés particulièrement préférés sont les suivants :
- le sel de potassium du sucrose octasulfaté ;
- le sel d'argent du sucrose octasulfaté ;
- le complexe hydroxyaluminium du sucrose octasulfate communément désigné par le nom sucralfate.

Le sel de potassium du sucrose octasulfate est connu depuis 25 ans pour le traitement des plaies lors de la phase de bourgeonnement grâce à son action sur les fibroblastes. Cette action est par exemple décrite dans les demandes de brevet EP 230 023, WO 89/05645 ou WO 98/22114 ou US 4912093. Comme il est précisé dans ces documents, ce composé est utilisé après avoir réalisé une détersion assistée de la plaie et donc après avoir éliminé les tissus nécrotiques et/ou fibrineux (voir EP 230 023, page 8, lignes 10 à 15 et exemple 18 ; page 33, lignes 28 à 30 ; voir WO 89/05645, page 25, lignes 8 et 9 ; et page 16, lignes 25 à 28 ; voir WO 98/22114, page 17, lignes 18 à 20). On l'utilise donc sur une plaie propre et détergée.

Dans la demande de brevet internationale WO 89/05645 (voir page 7 lignes 13 à 24), il est précisé que les polysaccharides polysulfatés tels que le sucralfate sont des inhibiteurs de protéases, notamment les hyaluronidases, qui sont des enzymes capables de dégrader l'acide hyaluronique et les glycosaminoglycanes.

Il est donc d'autant plus surprenant que ces composés puissent avoir une utilisation en détersion car les protéases sont des enzymes qui, au cours de la phase naturelle de détersion, permettent de favoriser la dégradation des tissus abîmés. Leur inhibition va donc à l'encontre d'une bonne détersion de la plaie.

Bien que pour l'instant le mécanisme par lequel les composés utilisés dans le cadre de la présente invention agissent soit inconnu, on a démontré qu'ils étaient capables de dégrader une matrice de fibrine reconstituée in vitro et on a pu vérifier cette propriété sur des prélèvements de tissus fibrineux réalisés in vivo.

D'une façon générale, ces composés pourront être utilisés seuls ou en mélange de deux ou plus d'entre eux, ou encore en combinaison avec une (ou plusieurs) autre(s) substance(s) active(s) permettant d'induire ou d'accélérer la cicatrisation ou pouvant avoir un rôle favorable dans le traitement de la plaie. De façon préférentielle, on utilisera ces composés avec des substances actives qui présentent un intérêt accru durant la phase de détersion. Parmi ces substances actives, on peut citer, en particulier, à titre d'exemples :
- les agents bactéricides ou bactériostatiques (chlorhexidine, sels ou complexes d'argent, sels de zinc ou de cuivre, métronidazole, néomycine) pour prévenir ou traiter les risques d'infections qui sont une des complications redoutées avec les tissus nécrosés ;
- les anesthésiques locaux (lidocaïne) ;
- les anti-inflammatoires comme les anti-inflammatoires non stéroïdiens (AINS) (ibuprofène, kétoprofène, diclofenac), les inhibiteurs de la cyclooxygénase 2 (celecoxib, rofecoxib) pour soulager la douleur qui accompagne souvent ces plaies ;
- les corticoïdes.

Bien entendu, les composés utilisés dans le cadre de la présente invention pourront aussi être utilisés avec un (ou plusieurs) autre(s) composé(s) connu(s) pour son (leur) action dans le cadre de la phase de détersion comme par exemple :
- des enzymes (ce qui permettrait ainsi d'utiliser ces dernières à de plus faibles concentrations et d'éviter leur problème de sensibilisation) ;
- l'urée.

Les composés utilisés dans le cadre de la présente invention pourront être mis en oeuvre simultanément ou successivement avec une autre substance active durant la phase de détersion en fonction de la nature (infection, niveau de nécrose, plaie douloureuse) ou de l'évolution (niveau de tissu fibrineux) de la plaie à déterger.

Dans le cadre de leur utilisation comme agent de détersion, les composés utilisés dans le cadre de la présente invention seront mis en oeuvre au sein d'une formulation galénique, comme par exemple un gel, une solution, une émulsion, une crème, des granules ou des capsules de taille variable allant du nano ou micromètre au millimètre, qui permettra leur application directement au niveau de la plaie. S'ils sont employés en mélange de deux ou plusieurs d'entre eux ou encore en combinaison avec une ou plusieurs autres substances actives, ces composés pourront être incorporés dans la même formulation galénique ou dans des formulations galéniques distinctes.

Alternativement, et de manière préférentielle, les composés utilisés dans le cadre de la présente invention ou une formulation galénique les contenant seront intégrés à un pansement.

Par pansement, on entend désigner ici tous types de pansements utilisés pour le traitement des plaies et, d'une façon préférée, les pansements absorbants car les plaies à traiter durant la phase de détersion sont souvent très exsudatives.

Par souci de simplicité, on désignera dans ce qui suit par l'expression "agent de détersion selon l'invention" tout composé ou mélange de composés choisi(s) parmi les oligosaccharides des sulfates décrits ci-dessus et dont l'utilisation est ici revendiquée.

L'agent de détersion selon l'invention ou une formulation galénique le contenant pourra être incorporé dans un élément quelconque de la structure d'un pansement.

De préférence et afin de favoriser une action rapide, ce composé (ou une formulation galénique le contenant) sera incorporé dans la couche du pansement qui vient en contact avec la plaie ou déposé sur la surface du pansement qui vient en contact avec la plaie.

De telles techniques de dépôt sont bien connues de l'homme de l'art et certaines sont par exemple décrites dans la demande de brevet WO 2006/007844.

Avantageusement, l'agent de détersion selon l'invention (ou une formulation galénique le contenant) pourra ainsi être déposé, de façon continue ou discontinue, sur la surface destinée à venir au contact de la plaie :
- soit sous forme liquide, par exemple par vaporisation d'une solution ou suspension le contenant ;
- soit sous forme solide, par exemple par tamisage d'une poudre le contenant.

La couche ou surface venant en contact avec la plaie pourra être constituée par exemple d'un matériau absorbant telle qu'une mousse absorbante hydrophile en polyuréthane ; un matériau textile telle qu'une compresse, comme par exemple un non tissé, un film, un matériau adhésif absorbant ou non ; une structure interface adhérente ou non.

De façon générale, on adaptera la galénique ou la structure du pansement pour obtenir un profil de relargage spécifique, rapide ou retardé, selon les besoins, de l'agent de détersion selon l'invention.

Bien entendu, la quantité d'agent de détersion selon l'invention utilisée dans la formulation galénique ou dans le pansement sera adaptée en fonction de la cinétique recherchée ainsi que des contraintes spécifiques liées à sa nature, solubilité, résistance à la chaleur, etc.

Ainsi, pour son utilisation dans une formulation galénique, l'agent de détersion selon l'invention pourra être incorporé en une quantité comprise entre 0,1 et 50% en poids rapportée au poids total de la formulation.

Dans le cadre de son utilisation dans un élément de pansement, l'agent de détersion selon l'invention sera incorporé en une quantité telle que la quantité de cet agent de détersion relarguée dans les exsudats de la plaie soit comprise entre 0,001 g/l et 50 g/l, et de préférence entre 0,001 et 10 g/l.

Dans un mode de réalisation préféré, l'agent de détersion selon l'invention sera incorporé dans un pansement absorbant à base de fibres gélifiantes comme par exemple le produit AQUACEL^{®} commercialisé par la société CONVATEC. On optimisera ainsi le pouvoir détersif de ce type de produit déjà utilisé dans le cadre de la détersion autolytique.

Selon une variante encore préférée, l'agent de détersion selon l'invention sera incorporé dans un pansement constitué d'un non tissé à base de fibres superabsorbantes qui gélifient au contact des exsudats de la plaie. De telles fibres sont par exemple commercialisées par la société TOYOBO Co Ltd sous la dénomination LANSEAL^{®} F.

Afin de renforcer l'intégrité de ces non tissés après absorption, ces fibres superabsorbantes pourront être associées, par exemple par aiguilletage ou thermoliage, à des fibres thermoliantes comme des fibres bicomposants constituées d'un coeur en polyester et d'une écorce en polyéthylène.

Des pansements non tissés pouvant être utilisés dans ce contexte sont par exemple décrits dans la demande de brevet internationale WO 2007/085391.

Très souvent, lors de la pose de ces pansements, le personnel soignant maintient ces derniers en place à l'aide d'une bande ou recouvre ces derniers d'un élément secondaire tel qu'un second pansement absorbant voire une bande de contention. Il est donc utile, que le pansement reste fixé sur la plaie afin que le personnel soignant conserve les mains libres pour poser ces éléments secondaires.

Dans le cadre de la présente invention, on utilisera donc de préférence un non tissé, tel que ceux cités précédemment, dans lequel la surface venant en contact avec la plaie est recouverte d'une couche non continue d'adhésif. Cette dernière pourra se présenter par exemple sous la forme de fibres d'adhésifs, d'une couche adhésive perforée, d'une couche discontinue d'adhésif sous forme de stries ou d'un filet ou de tout autre motif géométrique non continu.

D'une façon générale, tout type d'adhésif couramment employé dans les pansements pourra être utilisé à cet effet.

Afin de ne pas altérer les tissus sains ou les berges de la plaie, notamment lors du retrait du pansement, on préférera un adhésif ayant la propriété d'adhérer à la peau sans adhérer à la plaie.

A titre d'exemple d'un tel adhésif, on peut ainsi citer les adhésifs à base d'élastomères de silicone ou de polyuréthane, tels que les gels de silicone ou de polyuréthane et les adhésifs hydrocolloïdes.

De tels adhésifs hydrocolloïdes sont constitués d'une matrice élastomérique à base d'un ou plusieurs élastomères choisis parmi les polymères séquencés poly(styrène-oléfine-styrène) en association avec un ou plusieurs composés choisis parmi les plastifiants, tels que les huiles minérales, des résines tackifiantes et, si nécessaire, des antioxydants, dans laquelle est incorporée une quantité, de préférence faible, d'hydrocolloïde (3 à 20% en poids) comme par exemple la carboxyméthylcellulose de sodium ou des polymères superabsorbants comme les produits commercialisés sous la dénomination LUQUASORB^{®} par la société BASF.

La formulation de tels adhésifs hydrocolloïdes est bien connue de l'homme de l'art et décrite par exemple dans les demandes de brevet FR 2 392 076 et FR 2 495 473.

L'utilisation d'un filet d'adhésif sur le non tissé permet d'une façon particulièrement avantageuse de diminuer ou d'éviter le risque que de petites fibrilles du matériau textile viennent au contact de la plaie et s'accrochent aux tissus, en provoquant ainsi un phénomène de douleur au retrait, voire même un obstacle au processus de cicatrisation de la plaie. Elle permet en outre de mieux réguler le flux de liquide au niveau du matériau textile et de réduire ou d'éliminer les risques de "gel blocking", résultant de l'utilisation de fibres superabsorbantes qui limitent de fait la capacité d'absorption du non tissé.

Selon une variante de réalisation préférée de la présente invention, l'agent de détersion selon l'invention sera incorporé dans un tel adhésif à une concentration compatible avec sa solubilité et sa résistance à la chaleur.

Sur la base de ces critères, l'agent de détersion selon l'invention sera utilisé de préférence en une quantité comprise entre 1 et 15% en poids, et de préférence encore entre 5 et 10% en poids, rapportée au poids total de l'adhésif.

Si l'on souhaite augmenter l'absorption de ce pansement non tissé, on pourra associer ce dernier avec une couche absorbante additionnelle, et de préférence une couche absorbante qui ne gélifie pas, comme en particulier une mousse hydrophile absorbante, de préférence une mousse polyuréthane hydrophile présentant une capacité d'absorption supérieure à celle du non tissé.

De telles mousses et leur procédé de fabrication, notamment à partir de mélanges à base de prépolymère, eau, tensioactif... sont bien connues de l'homme de l'art et sont décrits par exemple dans les demandes de brevet suivantes : WO96/16099, WO 94/29361, WO 93/04101, EP 420 515, EP 392 788, EP 299 122, et WO 2004/074343.

Le non tissé et la mousse seront associés par des techniques bien connues de l'homme de l'art, par exemple par calandrage à chaud à l'aide d'une poudre thermofusible à base de polymères TPU/polycaprolactone. Cette technique est couramment employée pour le liage entre eux de non tissés destinés au marché médical.

Enfin, cette mousse ou le non tissé (lorsque celui-ci est utilisé seul) pourront être recouverts d'un support pour protéger la plaie de l'extérieur. Ce support pourra être de taille supérieure à celle des autres couches et adhésivé de façon continue ou discontinue sur sa face venant en contact avec la plaie afin d'optimiser le maintien du pansement lors de son usage, en particulier si la plaie se situe sur des zones corporelles non planes.

Ce support et son adhésif seront de préférence imperméables aux fluides mais très perméables à la vapeur d'eau afin de permettre une gestion optimale des exsudats absorbés par le pansement et éviter les problèmes de macération.

De tels supports sont bien connus de l'homme du métier et sont constitués par exemple de films respirants et imperméables tels que des films de polyuréthane, des complexes mousse/film ou non tissé/film.

Selon un autre aspect, la présente invention concerne une méthode de traitement des plaies qui comprend l'utilisation d'une composition selon l'invention qui comprend au moins un composé choisi parmi les oligosaccharides polysulfatés synthétiques ayant 1 à 4 unités oses, leurs sels et complexes.

Dans le cadre de cette méthode de traitement, cette composition est appliquée sur la plaie par tout moyen approprié et en particulier à l'aide d'un pansement tel que ceux décrits précédemment.

L'activité de l'agent de détersion selon l'invention a été mise en évidence dans différentes conditions :
- d'une part, sur des modèles de matrices de fibrine préparées in vitro ; et
- d'autre part, sur du tissu fibrineux prélevé sur des plaies.

Les essais qui ont été réalisés, et qui seront décrits de façon détaillée ci-après, ont ainsi démontré la capacité de ce composé à dégrader les matrices de fibrine et le tissu fibrineux. Ces essais ont également démontré, dans le modèle in vitro, la capacité à dégrader une matrice de fibrine d'un pansement utilisable dans le cadre de la détersion autolytique et constitué d'un non tissé à base de fibres gélifiantes, portant sur sa surface destinée à venir en contact avec la plaie, un filet d'adhésif hydrocolloïde incorporant l'agent de détersion selon l'invention.

Les résultats obtenus ont montré :
- d'une part, l'intérêt de l'agent de détersion selon l'invention dans le cadre de la détersion de la plaie ; et
- d'autre part, l'intérêt de l'utilisation de l'agent de détersion selon l'invention, dans le cadre de la détersion autolytique, où son incorporation dans un pansement absorbant, couramment utilisé dans cette technique, permet d'obtenir un produit optimal qui combine absorption des débris et action de dégradation du tissu fibrineux.

### Exemple 1 : Mise en évidence de l'effet du sel de potassium du sucrose octasulfate sur la dégradation de la matrice de fibrine préparée in vitro.

### 1. Réalisation de matrices de fibrine in vitro.

Les matrices de fibrine ont été préparées selon le protocole de Brown décrit dans la publication "Fibroblast migration in fibrin gel matrices" Arm J. Pathol, 1993, 142 : 273-283.

Les composants et le mode opératoire qui ont été utilisé sont les suivants :
On a solubilisé à 37°C :
   - 5 ml d'une solution aqueuse comprenant 50 millimoles d'Hépès (Catalogue Sigma-Aldrich)
   - 15 mg de fibrinogène de plasma humain (catalogue Sigma-Aldrich)
   - 5 millimoles de CaCl₂.

A la solution ainsi préparée, on a ajouté 50 µl de thrombine, 100 NIH de plasma humain (catalogue Sigma-Aldrich).

Le milieu réactionnel a été mélangé, déposé dans un tube de 15 ml (ou une boîte de Pétri de 60 mm de diamètre) puis laissé à incuber à 37°C.

Au cours des 24 premières heures d'incubation à 37°C, on a ainsi vu apparaître une matrice de fibrine qui a grossièrement l'aspect d'un gel.

Au bout de 24 heures, l'observation au microscope de cette matrice de fibrine, a montré la formation d'un réseau filamentaire homogène.

### 2. Mise en évidence de l'effet du sel de potassium du sucrose octasulfate sur la dégradation de la matrice de fibrine.

24 h après le début de la formation de la matrice de fibrine dans le tube de 15 ml précité, on a ajouté une solution de sel de potassium du sucrose octasulfate en une quantité de 1 volume de solution de ce composé pour 5 volumes du mélange fibrinogène/thrombine utilisé pour la préparation de la matrice de fibrine.

En parallèle, on a préparé une solution témoin exempte du composé testé avec un tampon phosphate salin (PBS, en abrégé : Phosphate Buffered Saline).

On a suivi visuellement la dégradation de la matrice de fibrine au cours des 24 et 48 heures suivantes.

Les observations ainsi réalisées ont été classées en 3 niveaux :
- Pas de dégradation : la matrice reste identique.
- Dégradation partielle : désagrégation de la matrice.
- Dégradation totale : la matrice disparaît.

### 3. Dosage des PDF :

Afin de quantifier et de vérifier que les observations réalisées correspondent bien à une dégradation de la matrice de fibrine, on a dosé les produits de dégradation de la fibrine (PDF) 24 heures ou 48 heures après l'ajout des solutions du composé testé

Ce dosage a été réalisé selon une technique classique en utilisant un kit de dosage PDF plasma de référence 00540 commercialisé par la société Diagnostica Stago.

On a ainsi prélevé 20 µlitres de surnageant dans le tube de 15 ml que l'on a déposé au centre de l'anneau rouge d'une plaque du kit de dosage PDF. On a ajouté 20 µlitres de suspension de particules de latex. Après agitation, on a réalisé l'analyse.

En corrélant les résultats visuels et les mesures de PDF, on a quantifié les résultats obtenus :
- si la valeur des PDF, exprimée en µg/ml, est inférieure à 5, il n'y a aucune dégradation de la matrice de fibrine,
- si la valeur des PDF, exprimée en µg/ml, est comprise entre 5 et 20, on a une dégradation partielle de la matrice de fibrine,
- si la valeur des PDF, exprimée en µg/ml, est supérieure à 20, on a une dégradation totale de la matrice de fibrine qui disparaît.

### 4. Tests avec le sel de potassium du sucrose octasulfate :

On a préparé une solution aqueuse de sel de potassium du sucrose octasulfate à 10 g/litres.

Cette solution a été testée selon le protocole décrit précédemment et on a mesuré les PDF 48 heures après l'ajout de la solution à la matrice de fibrine.

Afin d'avoir une évaluation de la pertinence des résultats, on a testé en témoin négatif une solution de PBS et en témoin positif le produit Accuzyme^{®} qui contient comme enzyme protéolytique, de la papaïne, associée à l'urée et qui est utilisée dans le cadre de la détersion enzymatique.

Par mesure des PDF, on a obtenu les résultats suivants :
- PBS : inférieur à 5 µg/ml
- Accuzyme^{®} : supérieur à 20 µg/ml
- Solution à 10g/l de sel de potassium du sucrose octasulfate : compris entre 5 et 20 µg/ml.

On a ainsi constaté l'efficacité du sel de potassium du sucrose octasulfate qui a entraîné une dégradation partielle de la matrice de fibrine.

### Exemple 2 : Mise en évidence de l'effet du sel de potassium du sucrose octasulfate sur du tissu fibrineux prélevé in vivo.

La même solution de sel de potassium du sucrose octasulfate a été testée sur du tissu fibrineux prélevé in vivo.

Pour réaliser ce test, dans un tube de 15 ml maintenu à 37°C, on a introduit un échantillon de tissu fibrineux issu d'un ulcère de jambe d'origine veineuse de 5 mm².

Au bout de 24 heures, on a déposé 1 ml de solution de sel de potassium du sucrose octasulfate sur cet échantillon.

On a préparé à nouveau un témoin négatif avec du PBS et un témoin positif avec Accuzyme^{®}.

On a dosé les PDF 48 heures après l'introduction de l'actif ou du témoin.

Cette expérience a été répétée sur 5 prélèvements de tissus fibrineux réalisés sur le même patient.

On a ainsi constaté que le sel de potassium du sucrose octasulfate a conduit, dans ce test, à une dégradation totale de la matrice de fibrine, démontrant ainsi son efficacité.

La mesure des PDF a confirmé ce résultat, les valeurs mesurées étant les suivantes :
- Accuzyme^{®} : supérieur à 20 µg/ml
- PBS : inférieur à 5 µg/ml
- Sel de potassium du sucrose octasulfate : supérieur à 20 µg/ml

Ces résultats confirment donc, sur du tissu fibrineux prélevé in vivo, les résultats obtenus sur le modèle in vitro, le niveau d'efficacité mesuré étant ici meilleur que sur le modèle de matrice de fibrine in vitro.

### Exemple 3 : Mise en évidence de l'effet du sel de potassium du sucrose octasulfate incorporé dans un pansement absorbant sur la dégradation de la matrice de fibrine préparée in vitro.

### 1. Préparation d'un pansement absorbant contenant le sel de potassium du sucrose octasulfate.

On a préparé par thermoliage un non tissé de 180 g/m² utilisable comme pansement absorbant dans le cadre de la détersion autolytique à l'aide de fibres superabsorbantes LANSEAL^{®} F commercialisées par la société TOYOBOCOLTD et de fibres thermoliantes bicomposants polyester/polyéthylène dans un ratio 70% (fibres superabsorbantes) /30% (fibres thermoliantes).

On a par ailleurs préparé un adhésif hydrocolloïde contenant le sel de potassium du sucrose octasulfate par mélange dans un malaxeur MEL G-40.

La composition de cet adhésif, exprimée en pourcentage en poids par rapport au poids total de l'adhésif était la suivante :
- Huile minérale commercialisée par la société Shell sous la dénomination Ondina ^{®}917 : 32,8%
- Sel de sodium de carboxyméthylcellulose (hydrocolloïde) commercialisé par la société AQUALON sous la dénomination CMC Blanose ^{®}7H4XF : 14%
- Sel de potassium du sucrose octasulfate commercialisé par la société EUTICALS : 7,5%
- Copolymère séquencé de poly(styrène-éthylène-butylène) (élastomère) commercialisé par la société KRATON sous la dénomination KRATON^{®} G 1654 : 6%
- Antioxydant commercialisé sous la dénomination IRGANOX^{®} 1010 par la société CIBA SPECIALTY CHEMICALS : 0,12%.
- Copolymère de sel de l'acide 2-méthyl-2[(1-oxo-2-propényl)amino]-1-propanesulfonique et de l'ester 2-hydroxyéthyle de l'acide propénoïque (agent de relargage) commercialisé par la société SEPPIC sous la dénomination SEPINOV^{®} EMT 10 : 5%.
- Résine tackifiante commercialisée par la société EXXON CHEMICALS sous la dénomination ESCOREZ^{®} 5380 : 35%.

On a introduit les différents constituants à une température comprise entre 100 et 110°C sous agitation, de manière à obtenir un mélange homogène.

Plus précisément, on a introduit initialement l'huile minérale, l' hydrocolloïde, le sel de potassium du sucrose octasulfate et l'élastomère puis l'antioxydant et l'agent de relargage et enfin la résine tackifiante.

Cet adhésif a été enduit de façon discontinue sous une configuration sous la forme d'un filet sur le non tissé préparé précédemment.

### 2. Tests des pansements sur le modèle de matrice de fibrine in vitro.

On a procédé ensuite à une comparaison entre le pansement contenant le sel de potassium du sucrose octasulfate et le pansement autolytique commercialisé par la société CONVATEC sous la dénomination AQUACEL^{®}.

A cet effet, on a préparé, suivant le protocole décrit précédemment, des matrices de fibrine dans des boîtes Petri de 60 mm de diamètre.

Au bout de 24 heures, on a déposé, à température ambiante, sur la matrice de fibrine, un échantillon de 20 mm de diamètre de chacun des 2 pansements décrits ci-dessus.

Les pansements ont été retirés 24 heures après leur pose et on a observé visuellement la matrice de fibrine.

On a ainsi constaté la formation d'un trou plus ou moins marqué, dans la matrice de fibrine, sous la surface correspondant à l'échantillon du pansement contenant le sel de potassium de sucrose octasulfate. A l'inverse, la matrice de fibrine est restée intacte pour l'échantillon du produit AQUACEL^{®}.

Cette expérience a donc confirmé l'efficacité du sel de potassium du sucrose octasulfate pour la dégradation de matrices de fibrine et son intérêt à l'incorporer dans un pansement utilisable dans le cadre de la détersion autolytique.

## Revendications

1. Composition comprenant au moins un composé choisi parmi les oligosaccharides polysulfatés synthétiques ayant 1 à 4 unités oses, leurs sels et complexes, pour son utilisation comme agent de détersion d'une plaie.

2. Composition pour son utilisation selon la revendication 1, **caractérisée en ce que** le composé précité est choisi parmi les oligosaccharides polysulfatés synthétiques ayant 1 ou 2 unités oses choisies de préférence parmi les pentoses et les hexoses, ainsi que les sels et complexes de ces composés.

3. Composition pour son utilisation selon la revendication 1 ou 2, **caractérisée en ce que** le composé précité est choisi parmi :
- le sel de potassium du sucrose octasulfaté ;
- le sel d'argent du sucrose octasulfaté ;
- le sucralfate.

4. Composition pour son utilisation selon l'une des revendications 1 à 3, **caractérisée en ce que** le composé précité est associé à une ou plusieurs autres substances actives permettant d'induire ou d'accélérer la cicatrisation ou pouvant avoir un rôle favorable dans le traitement de la plaie.

5. Composition pour son utilisation selon la revendication 4, **caractérisée en ce que** la substance active précitée est choisie parmi :
- les agents bactéricides ou bactériostatiques, comme en particulier la chlorhexidine, les sels ou complexes d'argent, les sels de zinc ou de cuivre, la métronisazole, la néomycine ;
- les anesthésiques locaux, comme en particulier la lidocaïne ;
- les anti-inflammatoires, comme en particulier les anti-inflammatoires non stéroïdiens et les inhibiteurs de la cyclooxygénase 2 ;
- les corticoïdes.

6. Composition pour son utilisation selon l'une quelconque des revendications 1 à 5, **caractérisée en ce que** le composé précité est associé à un ou plusieurs agents de détersion additionnels.

7. Composition pour son utilisation selon la revendication 6, **caractérisée en ce que** l'agent de détersion additionnel précité est choisi parmi :
- les enzymes ; et
- l'urée.

8. Composition pour son utilisation selon l'une quelconque des revendications 1 à 7, **caractérisée en ce que** le composé précité est mis en oeuvre au sein d'une formulation galénique, comme par exemple un gel, une solution, une émulsion, une crème, des granules ou des capsules permettant une application directement au niveau de la plaie, de préférence en une quantité comprise entre 0,1 et 50 % en poids, rapportée au poids total de la formulation.

9. Composition pour son utilisation selon l'une quelconque des revendications 1 à 8, **caractérisée en ce que** le composé précité ou une formulation galénique le contenant est intégré à un élément d'un pansement, de préférence en une quantité telle que la quantité de ce composé relarguée dans les exsudats de la plaie soit comprise entre 0,001 g/l et 50 g/l, et de préférence encore entre 0,001 et 10 g/l.

10. Composition pour son utilisation selon la revendication 9, **caractérisée en ce que** le pansement précité est un pansement à base de fibres absorbantes.

11. composition pour son utilisation selon la revendication 9, **caractérisée en ce que** le pansement précité est un pansement comprenant :
- un non tissé constitué de fibres superabsorbantes associées à des fibres thermoliantes dont la surface venant en contact avec la plaie est recouverte d'une couche non continue d'adhésif.

12. Composition pour son utilisation selon la revendication 11, **caractérisée en ce que** l'adhésif précité est un adhésif hydrocolloïde et **en ce que** l'oligosaccharide polysulfaté précité est incorporé dans ledit adhésif, de préférence en une quantité comprise entre 1 et 15 % en poids, de préférence encore entre 5 et 10 % en poids, rapportée au poids de l'adhésif.

## Patentansprüche

1. Zusammensetzung, umfassend mindestens eine Verbindung, die unter den synthetischen Polysulfat-Oligosacchariden mit 1 bis 4 Glykose-Einheiten, ihren Salzen und Komplexen ausgewählt ist, für ihre Verwendung als Mittel zur Wundreinigung.

2. Zusammensetzung zu Ihrer Verwendung nach Anspruch 1, **dadurch gekennzeichnet, dass** die vorgenannte Verbindung unter den synthetischen Polysulfat-Oligosacchariden mit 1 oder 2 Glykose-Einheiten, die vorzugsweise unter den Pentosen und den Hexosen ausgewählt sind, sowie den Salzen und komplexen dieser Verbindungen ausgewählt ist.

3. Zusammensetzung zu Ihrer Verwendung nach Anspruch 1 oder 2, **dadurch gekennzeichnet, dass** die vorgenannte Verbindung ausgewählt ist unter:
- dem Kaliumsalz der Octasulfat-Sucrose,
- dem Silbersalz der Octasulfat-Sucrose,
- dem Sucralfat.

4. Zusammensetzung zu Ihrer Verwendung nach einem der Ansprüche 1 bis 3, **dadurch gekennzeichnet, dass** die vorgenannte Verbindung mit einer oder mehreren weiteren Aktivsubstanzen verbunden ist, die es ermöglichen die Narbenbildung hervorzurufen oder zu beschleunigen, oder die eine günstige Rolle bei der Wundbehandlung spielen können.

5. Zusammensetzung zu Ihrer Verwendung nach Anspruch 4, **dadurch gekennzeichnet, dass** die vorgenannte Aktivsubstanz ausgewählt ist unter:
- den bakteriziden oder bakteriostatischen Wirkstoffen, wie insbesondere dem Chlorhexidin, den Silbersalzen oder -komplexen, den Zink- oder Kupfersalzen, dem Metronisazol, dem Neomycin,
- den Lokalanästhetika, wie insbesondere Lidocain,
- den Entzündungshemmern, wie insbesondere den nicht steroiden Entzündungshemmern und den Cyclooxygenase 2-Hemmern,
- den Corticoiden.

6. Zusammensetzung zu Ihrer Verwendung nach einem der Ansprüche 1 bis 5, **dadurch gekennzeichnet, dass** die vorgenannte Verbindung mit einem oder mehreren zusätzlichen Wundreinigungsmitteln verbunden ist.

7. Zusammensetzung zu Ihrer Verwendung nach Anspruch 6, **dadurch gekennzeichnet, dass** das vorgenannte zusätzliche Wundreinigungsmittel ausgewählt ist unter:
- den Enzymen und
- dem Harnstoff.

8. Zusammensetzung zu Ihrer Verwendung nach einem der Ansprüche 1 bis 7, **dadurch gekennzeichnet, dass** die vorgenannte Verbindung in einer galenischen Formel, wie beispielsweise einem Gel, einer Lösung, einer Emulsion, einer Creme, Granulat oder Kapseln eingesetzt wird, die ein Auftragen direkt auf die Wunde vorzugsweise in einer Menge zwischen 0,1 und 50 Gew.-% bezogen auf das Gesamtgewicht der Formel ermöglichen.

9. Zusammensetzung zu Ihrer Verwendung nach einem der Ansprüche 1 bis 8, **dadurch gekennzeichnet, dass** die vorgenannte Verbindung oder sie enthaltende galenische Formel in ein Element eines Verbandes vorzugsweise in einer derartigen Menge integriert ist, dass die Menge dieser Verbindung, die in die Exsudate der Wunde freigesetzt wird, zwischen 0,001 g/l und 50 g/l und vorzugsweise zwischen 0,001 und 10 g/l, beträgt.

10. Zusammensetzung zu Ihrer Verwendung nach Anspruch 9, **dadurch gekennzeichnet, dass** der vorgenannte Verband auf Basis von absorbierenden Fasern ist.

11. Zusammensetzung zu Ihrer Verwendung nach Anspruch 9, **dadurch gekennzeichnet, dass** der vorgenannte Verband ein Verband ist, umfassend:
- ein Vlies, das von superabsorbierenden Fasern in Verbindung mit wärmeverbindbaren Fasern gebildet ist, dessen Fläche, die mit der Wunde in Kontakt kommt, mit einer nicht durchgehenden Haftschicht bedeckt ist.

12. Zusammensetzung zu Ihrer Verwendung nach Anspruch 11, **dadurch gekennzeichnet, dass** das vorgenannte Haftmittel ein Hydrokolloid-Haftmittel ist, und dass das vorgenannte Polysulfat-Oligosaccharid in das Haftmittel integriert ist, vorzugsweise in einer Menge zwischen 1 und 15 Gew.-%, noch bevorzugter zwischen 5 und 10 Gew.-% bezogen auf das Gewicht des Haftmittels.

## Claims

1. A composition comprising at least one compound selected from synthetic polysulfated oligosaccharides having 1 to 4 monosaccharide units, salts and complexes thereof, for use as a wound cleaning agent.

2. The composition for its use according to claim 1, **characterized in that** the aforementioned compound is selected from synthetic polysulfated oligosaccharides having 1 or 2 monosaccharide units preferably selected from pentoses and hexoses, as well as salts and complexes of these compounds.

3. The composition for its use according to claim 1 or 2, **characterized in that** the aforementioned compound is selected from:
- the potassium salt of sucrose octasulfate;
- the silver salt of sucrose octasulfate;
- sucralfate.

4. The composition for its use according to one of claims 1 to 3, **characterized in that** the aforementioned compound is combined with one or more other active substances for inducing or accelerating healing or that can have a favorable role in wound treatment.

5. The composition for its use according to claim 4, **characterized in that** the aforementioned active substance is selected from:
- bactericidal or bacteriostatic agents, such as in particular chlorhexidine, silver salts or complexes, zinc salts or copper salts, metronisazole, neomycin;
- local anesthetics, such as in particular lidocaine;
- anti-inflammatories, such as in particular nonsteroidal anti-inflammatories and cyclooxygenase-2 inhibitors;
- corticoids.

6. The composition for its use according to any one of claims 1 to 5, **characterized in that** the aforementioned compound is combined with one or more additional wound cleaning agents.

7. The composition for its use according to claim 6, **characterized in that** the aforementioned additional wound cleaning agent is selected from:
- enzymes; and
- urea.

8. The composition for its use according to any one of claims 1 to 7, **characterized in that** the aforementioned compound is used in a pharmaceutical formulation, for example a gel, a solution, an emulsion, a cream, granules or capsules permitting application directly on the wound, preferably in an amount between 0.1 and 50 wt.%, relative to the total weight of the formulation.

9. The composition for its use according to any one of claims 1 to 8, **characterized in that** the aforementioned compound or a pharmaceutical formulation containing it is integrated with an element of a dressing, preferably in an amount such that the amount of this compound released in wound exudates is between 0.001 g/l and 50 g/l, and more preferably between 0.001 and 10 g/l.

10. The composition for its use according to claim 9, **characterized in that** the aforementioned dressing is a dressing based on absorbent fibers.

11. The composition for its use according to claim 9, **characterized in that** the aforementioned dressing is a dressing comprising:
- a nonwoven consisting of superabsorbent fibers combined with thermal-bonding fibers whose surface coming in contact with the wound is covered with a discontinuous layer of adhesive.

12. The composition for its use according to claim 11, **characterized in that** the aforementioned adhesive is a hydrocolloid adhesive and **in that** the aforementioned polysulfated oligosaccharide is incorporated in said adhesive, preferably in an amount between 1 and 15 wt.%, more preferably between 5 and 10 wt.%, relative to the weight of the adhesive.
